# EUROPEAN PATENT SPECIFICATION

(11) **EP 4 384 088 B1**
(45) Date of publication and mention of the grant of the patent: **11.03.2026**
(21) Application number: 22750736.5
(22) Date of filing: 03.08.2022
(51) Int. Cl.: A61B 17/16

(54) **ADJUSTABLE MILLING CUTTER**
VERSTELLBARER FRÄSER
FRAISE RÉGLABLE

(30) Priority: 10.08.2021 IT 202100021674
(43) Date of publication of application: 19.06.2024
(73) Proprietor: Melozzi, Alessandro, 64100 Teramo (TE) (IT)
(72) Inventor: Melozzi, Alessandro, 64100 Teramo (TE) (IT)
(74) Representative: Cutropia, Gianluigi
(86) International application number: PCT/IB2022/057221
(87) International publication number: WO 2023/017368

(56) References cited:
- US-A1- 2006 025 774
- US-A1- 2006 217 730

## Description

The present invention relates to an adjustable milling cutter particularly used for bone milling procedures to create a site in the bone suitable for accommodating an implant.

Although specific reference is hereinafter made to the medical and orthopedic field of activity and to a hip prosthetic implant, the milling cutter according to the invention can be also used to enlarge/create other types of implant sites in bones or for other uses in fields of activity other than the medical and orthopedic one.

As it is well known, a hip prosthesis includes:
- an acetabular cup fixed to the patient's acetabulum,
- a ball head coupled in the acetabular cup so as to obtain a ball joint, and
- a stem connected to the spherical head and fixed to the femur.

An acetabular site suitable for accommodating the acetabular cup is prepared in the acetabulum. The acetabular site is prepared during a bone surgery operation using a rotary milling cutter.

DE2500958A1 discloses a milling tool for the preparation of an acetabular site having a semi-spherical head with projections, which is driven into rotation by means of an electric motor, so as to mill the bone to obtain the site.

A plurality of heads with a different size is commercially available, which are classified according to the maximum diameter of the head. By way of example, ten sizes of heads are known, ranging from a minimum diameter of 42 mm to a maximum diameter of 52 mm, differing from each other by 1 mm.

Based on the patient's bone configuration and on the type of site to be prepared, the surgeon will choose the head size to be used and to be mounted on the milling cutter.

Sometimes during a surgical procedure, the surgeon starts with a small head in order to make an initial hole and gradually enlarges the hole using a bigger head.

So, the surgeon must have all sizes of heads available, thus causing a complicated management of the stocks of surgical instruments.

In addition, the replacement of a head in the milling cutter is inconvenient, time-consuming, and complex.

US2006/217730A1 discloses an expandable spring loaded acetabular reamer, comprising a plurality of convex reaming segments arranged symmetrically in pairs around a central core of the tool.

The purpose of the present invention is to eliminate the drawbacks of the prior art by providing an adjustable milling cutter that is versatile and adaptable to any type of surgical operation.

Another purpose is to provide such an adjustable milling cutter that is practical and easy for the operator to use.

Still another purpose is to provide such an adjustable milling cutter that is reliable and safe.

These purposes are achieved in accordance with the invention with the features of the appended independent claim 1.

Advantageous achievements of the invention appear from the dependent claims.

Further features of the invention will appear clearer from the following detailed description, which refers to merely illustrative and therefore nonlimiting embodiments, illustrated in the appended drawings, wherein:
Fig. 1 is an exploded perspective view illustrating the various components of a milling cutter according to the invention;
Figs. 1A and 1B are two enlarged views of details of Fig. 1;
Fig. 2 is a perspective view of the assembled milling cutter of Fig. 1;
Fig. 3 is a side view of a shaft of the milling cutter according to the invention;
Fig. 4 is an axial sectional view of the shaft along the sectional plane IV-IV of Fig. 3;
Fig. 5 is a perspective view of a stem of the milling cutter according to the invention;
Fig. 6 is an axial view of the stem of Fig. 5;
Fig. 7 is an axial view of a sleeve of the milling cutter according to the invention;
Fig. 8 is a front view of a cam of the milling cutter according to the invention;
Fig. 9 is an axial sectional view of the cam according to the sectional plane IX-IX of Fig. 8;
Fig. 10 is a front view of a metal ring of the milling cutter according to the invention;
Fig. 11 is an axial view of the metal ring of Fig. 10;
Fig. 12 is an axial view of the assembled milling cutter according to the invention;
Fig. 12A is an enlarged detail of Fig. 12;
Fig. 12B is an enlarged detail of Fig. 12A;
Fig. 13 is a cross-sectional view of the milling cutter according to the invention, taken along the sectional plane XIII-XIII of Fig. 12;
Fig. 14 is a cross-sectional view taken along the sectional plane XIV-XIV of Fig. 12A, illustrating an indexed rotation mechanism between the metal ring and the shaft;
Fig. 15 is an exploded perspective view of a second embodiment of the milling cutter according to the invention;
Fig. 15A is an enlarged detail of Fig. 15;
Fig. 16 is a side view of the assembled milling cutter of Fig. 15;
Fig. 17 is an axial view taken along the sectional plane XVII-XVII of Fig. 16;
Fig. 17A is an enlarged detail of Fig. 17;
Fig. 18 is a perspective view of a head portion of the milling cutter of Fig. 15;
Fig. 19 is an axial view illustrating three components of the milling cutter of Fig. 15;
Fig. 20 is an exploded perspective view of a head portion, illustrating a variant of the milling cutter of Fig. 15;
Fig. 21 is a side view of the head portion of the milling cutter of Fig. 20 in assembled condition;
Fig. 22 is a perspective view of the head portion of the milling cutter of Fig. 20 in assembled condition; and
Fig. 23 is a rear view of the variant of Fig. 22.

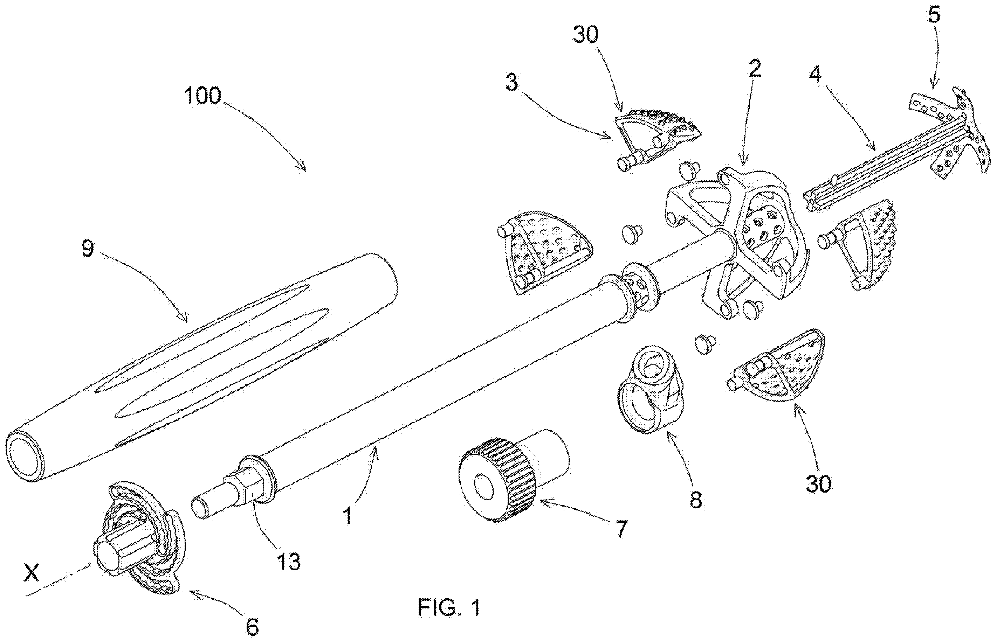

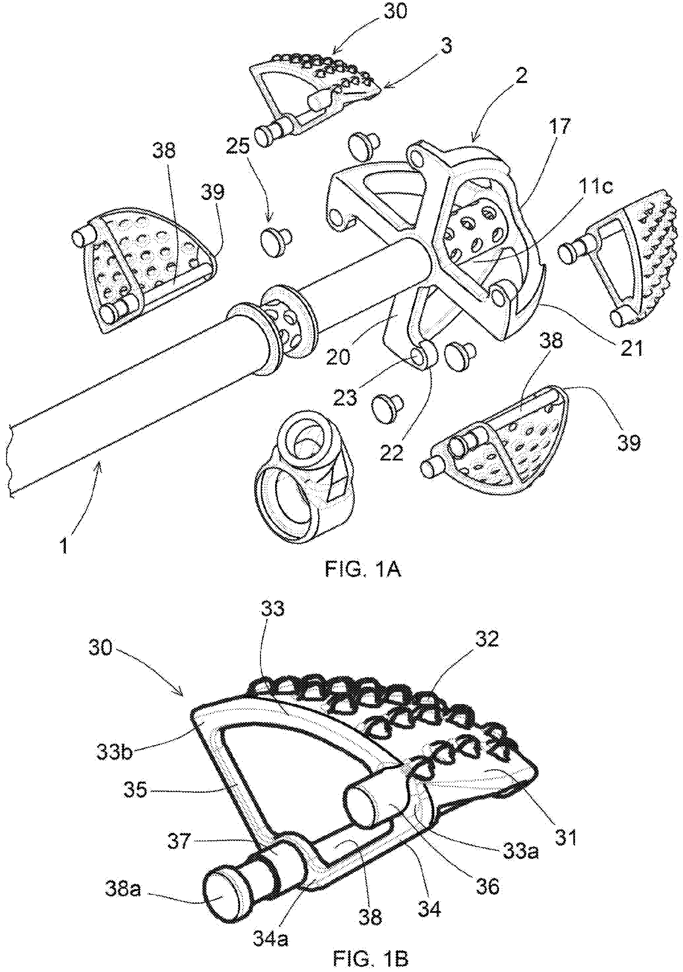

With the aid of Figs. 1 to 14, a milling cutter is described according to the invention, which is collectively indicated by reference numeral 100.

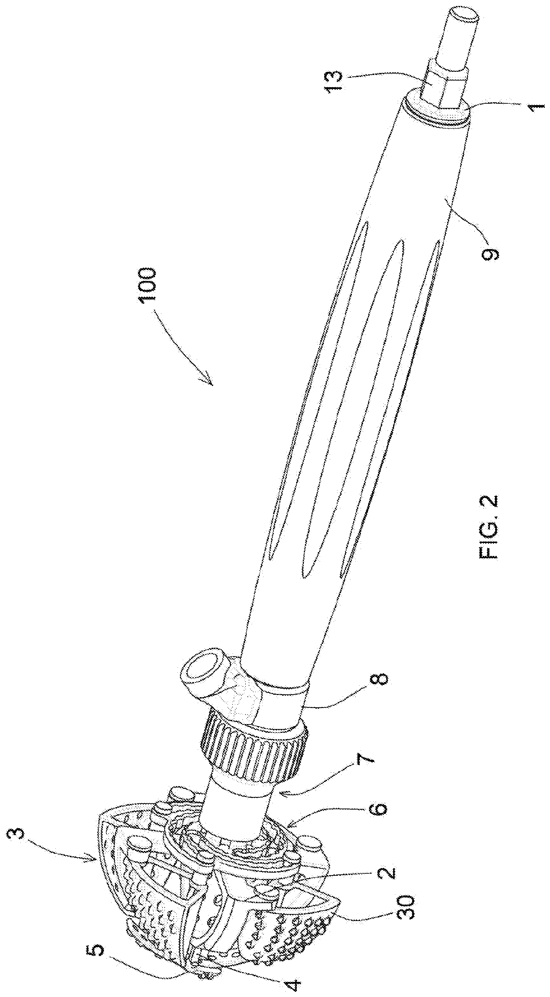

Now with reference to Figs. 1 and 2, the milling cutter (100) comprises:
- a shaft (1) comprising a frame (2),
- a head (3) in the shape of a spherical cap comprising a plurality of portions (30) mounted on the frame (2),
- a stem (4) comprising a tip (5) arranged between the portions (30) of the head,
- a cam (6) movably mounted on the shaft (1) and connected to the portions (30) of the head, and
- a metal ring (7) rotatably mounted on the shaft (1) and connected to the stem (4) and to the cam (6).

The portions (30) of the head are movably mounted on the frame (2) so as to move radially relative to the shaft (1) from a position of minimum head diameter to a position of maximum head diameter.

The expression "head diameter" means the maximum head diameter, that is to say the diameter taken at the base of the head that defines the maximum diameter of the site to be made in the bone.

The stem (4) is mounted in the shaft (1) with possibility of sliding axially, so that the tip (5) of the stem can move from a retracted position to an advanced position.

In this way, a clockwise/counterclockwise rotation of the metal ring (7) causes an increase/decrease in the diameter of the head (3) and an advancement/retraction of the tip (5) of the stem.

Optionally, the milling cutter (100) has a coupling (8) mounted on the shaft (1) for connection to an air intake system and a sleeve (9) mounted on the stem to act as a handle for the operator.

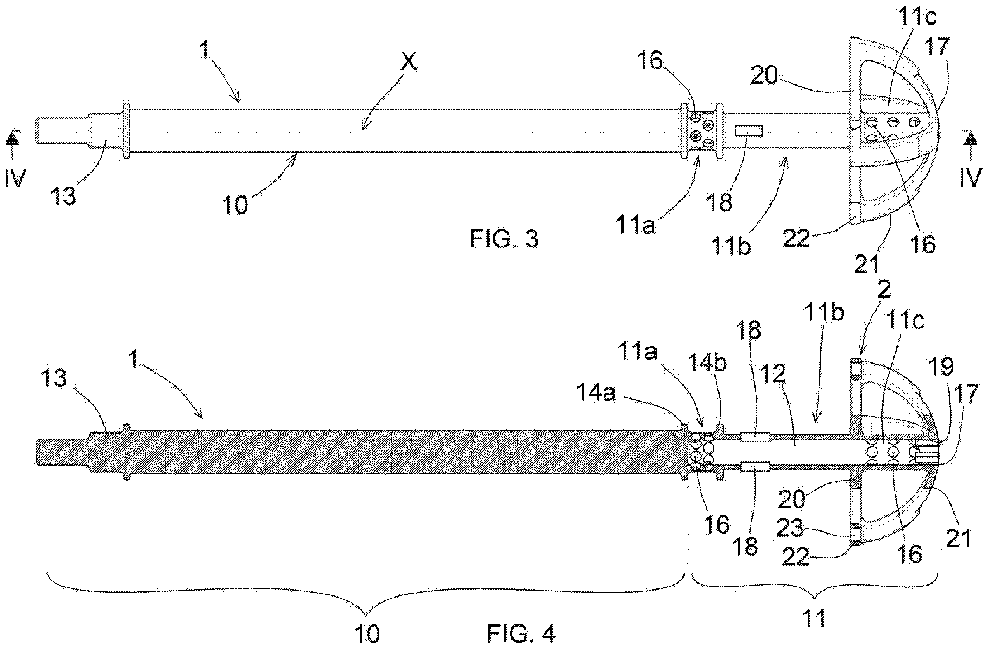

Referring to Figs. 3 and 4, the shaft (1) has a cylindrical body having an X axis that will be referred to as the axis of the milling cutter.

The shaft (1) has a solid rear portion (10) and an internally hollow front portion (11). The front portion (11) of the shaft has an axial channel (12). The rear portion (10) of the shaft is about 2-3 times longer than the front portion (11) of the shaft.

A coupling (13) is provided at a rear end of the shaft for connection to an electric motor in order to rotate the shaft (1) around the axis (X) of the milling cutter.

The front portion (11) of the shaft includes a perforated rear portion (11a), an intermediate portion (11b) and a perforated front portion (11c). The perforated rear and front portions (11a, 11c) have radial holes (16) communicating with the axial channel (12).

The perforated rear portion (11a) is defined between two collars (14a, 14b). In this way, the coupling (8) is mounted on the perforated rear portion (11a), between the two collars (14a, 14b).

Referring to Fig. 1A, the frame (2) is provided in the perforated front portion (11c) of the shaft. The frame (2) is cross-shaped and includes four brackets (20) that protrude radially from the front portion of the shaft and are arranged in orthogonal position. Each bracket (20) is connected to a front end (17) of the shaft by means of an arc-shaped arcuated rod (21). The arcuated rods (21) are located above the perforated front portion (11c).

Each bracket (20) has a flange (22) that protrudes from one end of the bracket so that the frame has a swastika shape when viewed from the rear. Each flange (22) has a through hole (23).

The head (3) comprises four identical portions (30).

Referring to Fig. 1B, each portion (30) of the head has a side wall (31) in the shape of a spherical cap portion. A plurality of abrasive projections (32) suitable for milling the bone protrudes outwardly from the side wall (31).

Each side wall (31) of the head portion has a posterior edge (33), in the shape of an arc subtended by a 90° angle. The posterior edge (33) of the side wall has a first end (33a) connected to a first rod (34)

A first pivoting pin (36) protrudes posteriorly from the first end (33a) of the rear edge of the side wall of the head portion in such a way to be pivoted into the hole (23) of the flange (22) of the frame of the shaft.

A first rod (34) protrudes radially from the first end (33a) of the rear edge of the side wall of the head portion.

A second pivoting pin (37) protrudes posteriorly from one end (34a) of the first rod (34) in such a way to be pivoted to the cam (6).

A second rod (35) protrudes radially from the second end (33b) of the rear edge of the side wall of the head portion and is connected to the end (34a) of the first rod.

A stiffening shaft (38) is connected to the second pivoting pin (37) and to a front end (39) of the head portion.

The two rods (34, 35) are arranged at 90°. In this way, the head portion (30) has a base in the shape of a 90° sector of a circle.

The first pivoting pin (36) and the second pivoting pin (37) are hollow tangs. The stiffening shaft (38) is mounted in the second pivoting pin (37) and is fixed to the front end (39) of the side wall (31) of the head portion. The stiffening shaft (38) has a head (38a) that protrudes posteriorly from the second pivoting pin (37).

The first pivoting pin (36) of each head portion is pivoted inside the hole (23) of the flange (22) of the frame (2). A locking pin (25) is inserted into the hole (23) of the flange (22) of the frame (2) to block a translation of the first pivoting pin (36). In this way, the stiffening shaft (38) is parallel to the axis (X) of the milling cutter.

In the condition in which the head (3) has a minimum diameter, the stiffening shaft (38) of each head portion is close to the respective bracket (20) of the frame. By moving the stiffening shaft (38) of each head portion away from the respective bracket (20) of the frame, the first pivoting pin (36) of the head portion can rotate in the hole (23) of the flange of the frame, and thus the second end (33b) of the rear edge of the head portion is radially moved away from the axis (X) of the milling cutter, increasing the diameter of the head (3).

Referring to Fig. 4, two longitudinal slots (18) are obtained at diametrically opposite positions in the intermediate portion (11b) of the front portion (11) of the shaft.

The axial channel (12) of the shaft has a female grooved portion (19) disposed at a front end of the shaft. The female grooved portion (19) has longitudinal ribs and grooves.

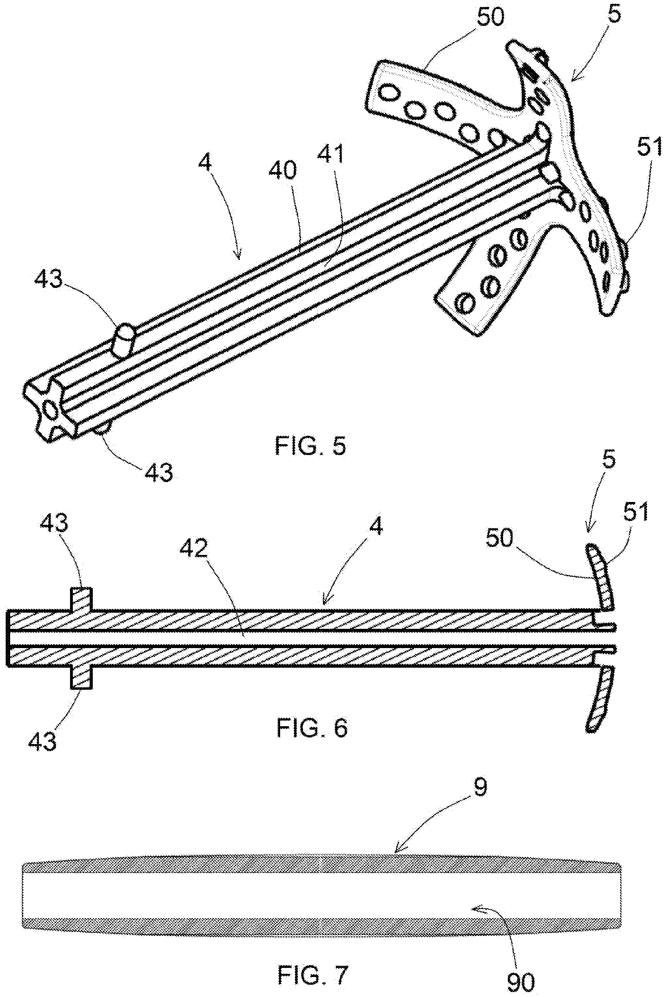

Referring to Figs. 5 and 6, the stem (4) has a grooved shape including longitudinal ribs (40) and longitudinal grooves (41). Thus, the stem (4) engages smoothly in the grooved profile (19) of the axial channel of the shaft, so that it can slide in the axial channel (12) of the shaft, without rotating. As an example, the stem (4) has a cross-section in the shape of a cross, with four longitudinal ribs and four longitudinal grooves.

The tip (5) of the stem includes four arcuate plates (50) that protrude radially from the stem and are arranged between the portions (30) of the head. Each arcuate plate (50) of the tip of the stem has abrasive projections (51) that are used to mill the bone.

The stem (4) has an axial channel (42) that is open anteriorly and posteriorly to extract air from the tip (5) of the stem.

Two pins (43) protrude radially from the stem (4), in diametrically opposite positions.

The pins (43) engage the longitudinal slots (18) of the shaft. The length of the longitudinal slots (18) and the diameter of the pins (43) are selected so that the stem (4) can make a preset translation travel, such as for example a translation travel of 5 mm. In this way, the tip (5) of the stem can be gradually extracted in such a way to adjust to the diameter increase of the head (3).

With reference to Fig. 7, the sleeve (9) is internally hollow and has an axial channel (90) wherein the front portion (10) of the shaft is arranged.

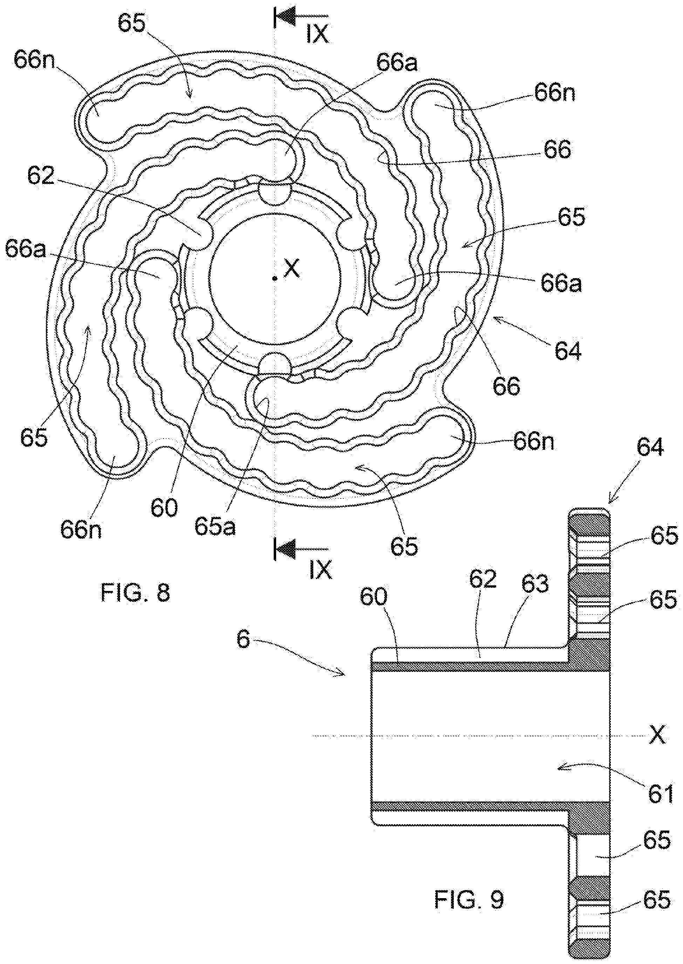

Referring to Figs. 8 and 9, the cam (6) comprises an internally hollow cylindrical body (60) having an axial channel (61) with an axis coincident with the axis (X) of the milling cutter. In this way, the cam (6) can be keyed on the shaft (1). The cam (6) is placed in the intermediate portion (11b) of the front portion of the shaft, abutting against the frame (2).

The cylindrical body (60) of the cam has a grooved outer surface with longitudinal grooves (62) and longitudinal ribs (63).

A plate (64), in the shape of a pinwheel, with an axis coincident with the axis (X) of the milling cutter, protrudes radially from a front edge of the cylindrical body (60) of the cam. The plate (64) is arranged along a plane orthogonal to the axis (X) of the milling cutter and has a center on the axis (X) of the milling cutter.

The plate (64) includes four slots (65) in the shape of an arc of a circle, e.g., in the shape of a quarter of circumference.

Each slot (65) includes a plurality of housings (66), arranged sequentially from a proximal housing (66a) close to the cylindrical body (60) of the cam to a distal housing (66n) distant from the cylindrical body (60) of the cam. The housings (66) have a rounded shape with a center.

The proximal housings (66a) are angularly equidistant near the cylindrical body (60). Likewise, the distal housings (66n) are arranged at the same radial distance from the axis of the cylindrical body (60) of the cam and are angularly equidistant.

The housings (66) are configured so that the radial distance between the axis (X) of the milling cutter and the center of the housing increases by a fixed amount, e.g., 1mm for each housing, going from the proximal housing (66a) to the distal housing (66n).

By way of example, ten housings (66) are provided in each slot (65) of the cam. Therefore, the difference between the radial distance between the axis (X) of the milling cutter and the center of the distal housing and the radial distance between the axis (X) of the milling cutter and the center of the proximal housing is 10 mm.

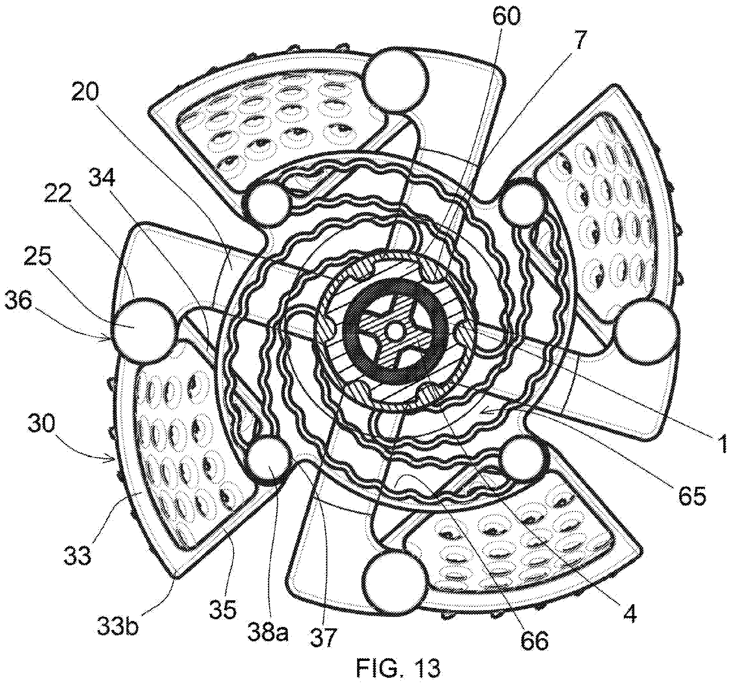

Referring to Fig. 13, the second pivoting pin (37) of each head portion is inserted into a housing (66) of each slot (65) of the plate of the cam. Fig. 13 illustrates the situation where the second pivoting pin (37) of each head portion is inserted into the distal housing (66n) of each slot (65) of the plate of the cam. In such a situation, the second end (33b) of the rear edge (33) of the side wall of the head portion is at a maximum radial distance from the axis (X) of the milling cutter and thus the head (3) has a maximum diameter.

By sliding the second pivoting pin (37) into the housings (66) of the slots of the cam, the second end (33b) of the rear edge (33) of the side wall of the head portion gradually approaches the axis (X) of the milling cutter, and thus the head (3) decreases its diameter.

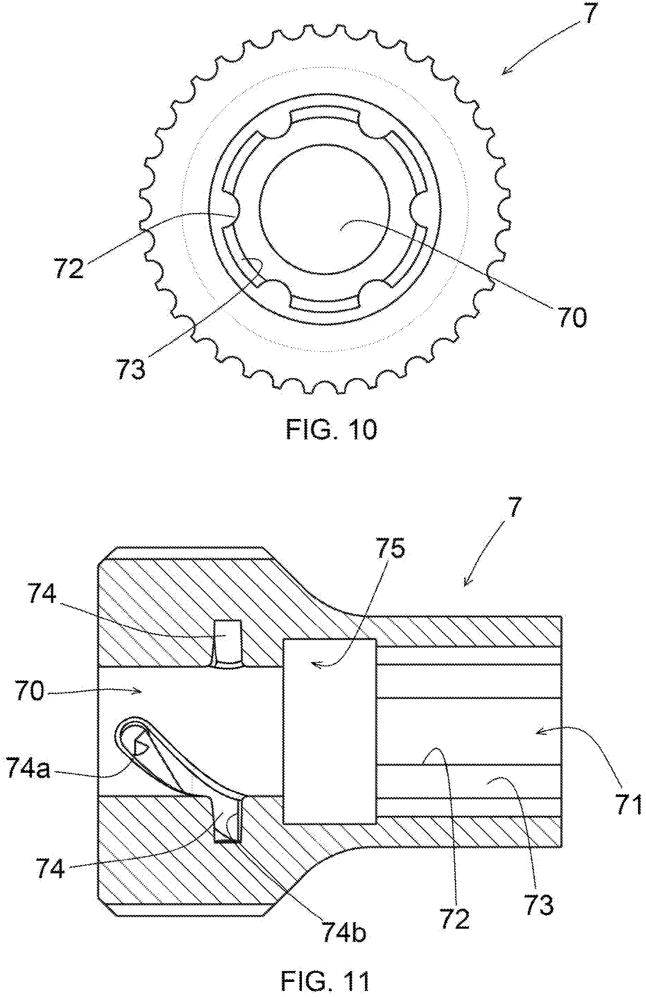

With reference to Figs. 10 and 11, the metal ring (7) is internally hollow and has an axial channel (70) in order to be keyed onto the shaft (1).

The axial channel (70) of the metal ring has a grooved portion (71) arranged anteriorly. The grooved portion (71) comprises longitudinal ribs (72) and longitudinal grooves (73) suitable for being coupled with the longitudinal grooves (62) and longitudinal ribs (63) of the cylindrical body (60) of the cam, in grooved coupling mode. Thus, a rotation of the metal ring (7) results in a rotation of the cam (6).

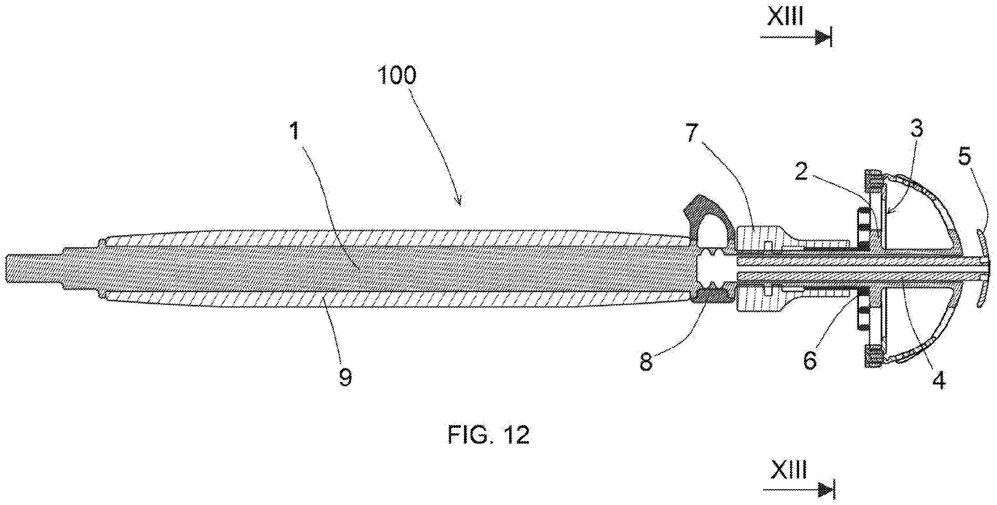

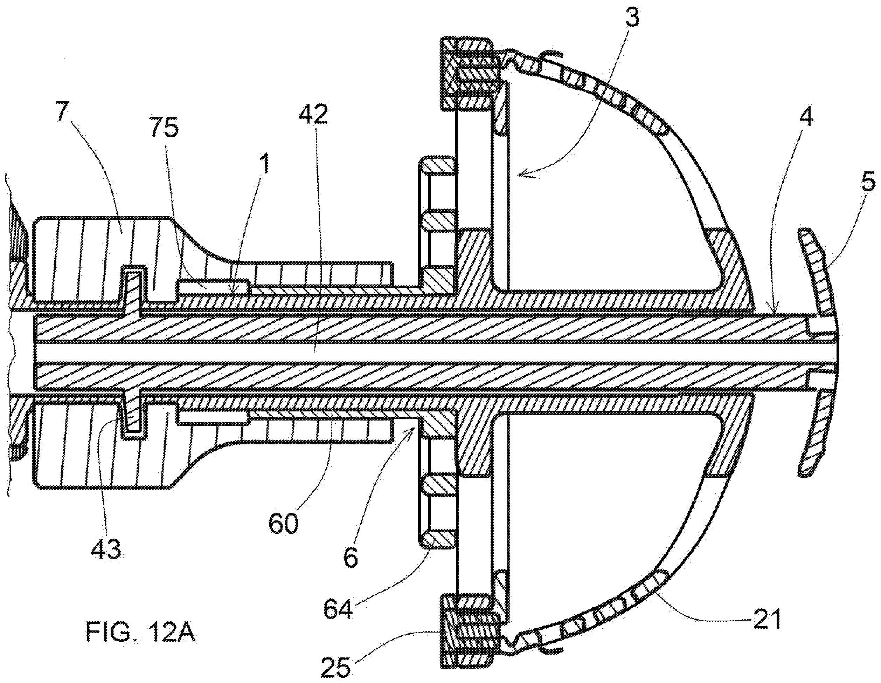

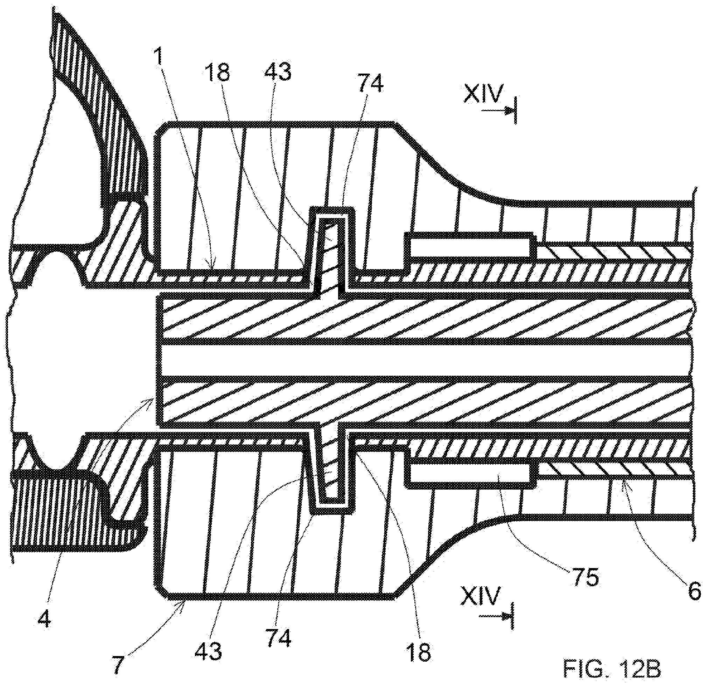

Two cam grooves (74) are cut in diametrically opposite positions in a rear part of the axial channel (70) of the metal ring. Each cam groove (74) has a helical, thread-like shape, extending from a rear end (74a) to a front end (74b). As shown in Fig. 12B, the cam grooves (74) of the metal ring are arranged at the longitudinal slots (18) of the shaft, so that they are engaged by the pins (43) of the stem that pass through the longitudinal slots (18) of the shaft.

In view of the above, by rotating the metal ring (7), the pins (43) of the stem slide in the cam grooves (74) of the metal ring and are pushed forward or backward, depending on the direction of rotation of the metal ring. As a result, the stem (4) translates, without rotating, according to the direction of rotation of the metal ring (7).

The metal ring (7) includes an indexing mechanism (75) that is coupled with the shaft (1) to allow an indexed step-by-step rotation of the metal ring (7) relative to the shaft (1).

The indexing mechanism (75) provides a number of clicks equal to the number of housings (66) of the cam. Thus, with each click of the indexing mechanism (75), the second pivoting pin (37) of the head portion enters a housing (66) of the slot of the cam.

As an example, the indexing mechanism (75) allows ten clicks, with a rotation of 13.5° between each click. In this way, the metal ring (7) has a rotation travel of 135°. By turning the metal ring (7) clockwise, the head diameter increases; by turning the metal ring (7) counterclockwise, the head diameter decreases.

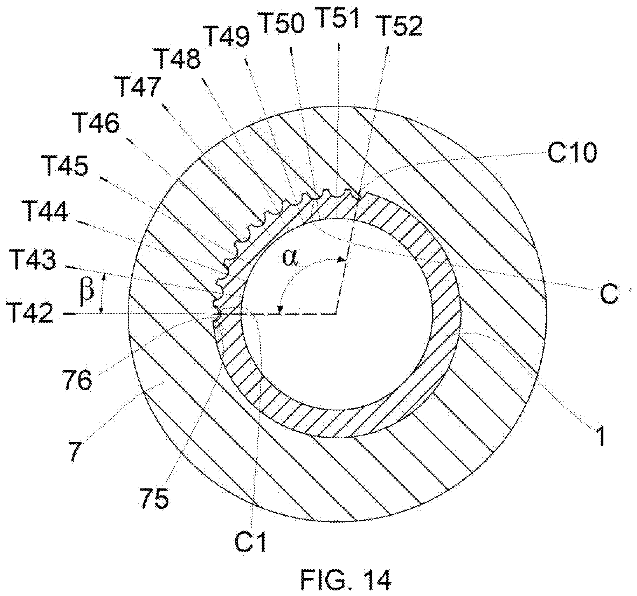

Fig. 14 illustrates a possible embodiment of the indexing mechanism (75). The indexing mechanism (75) includes a protrusion (76) integral with the metal ring (7) that projects radially inward into the axial channel of the metal ring.

The shaft (1) includes a plurality of cavities (C) arranged on an outer surface of the shaft, along an arc of circumference subtended by an angle (α) of 135°, at the protrusion (76) of the metal ring. In this way, the protrusion (76) of the metal ring can be sequentially snapped in the cavities (C) of the shaft.

In a clockwise direction, the cavities (C) go from a first cavity (C1) to a last cavity (C10). For example, ten cavities (C) that are angularly equidistant by an angle (β) equal to 13.5° are provided.

When the protrusion (76) of the metal ring is in the first cavity (C1), the head (3) has a minimum diameter that, for example, corresponds to a size (T42) of 42 mm. When the protrusion (76) of the metal ring is in the last cavity (C10), the head (3) has a maximum diameter that for example corresponds to a size (T52) of 52 mm. With each click of the metal ring (7), the protrusion (76) of the metal ring passes into the next cavity and the diameter of the head (3) increases by 1 mm.

In addition, the cam groove (74) of the metal ring is configured with a thread pitch such that for a 13.5° rotation of the metal ring clockwise, a 0.5 mm advancement of the stem (4) is achieved. In this way, for each click of the metal ring (7) there is an increase in the diameter of the head by 1 mm and an advancement of the tip (5) of the stem by 0.5 mm, so that the tip (5) of the stem can adjust to the increase in diameter of the head (3).

Hereafter, elements that are identical or corresponding to those already described are given the same reference numerals, omitting their detailed description.

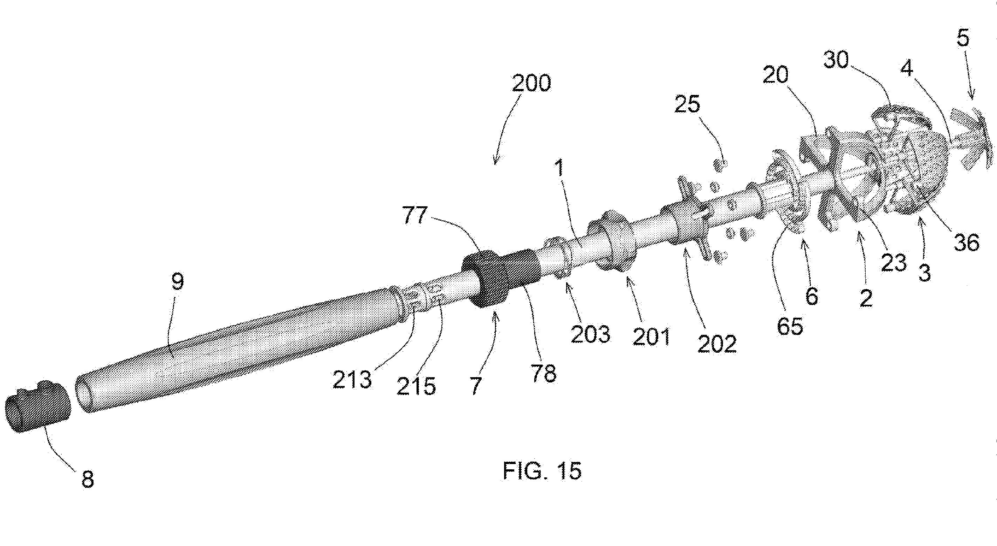

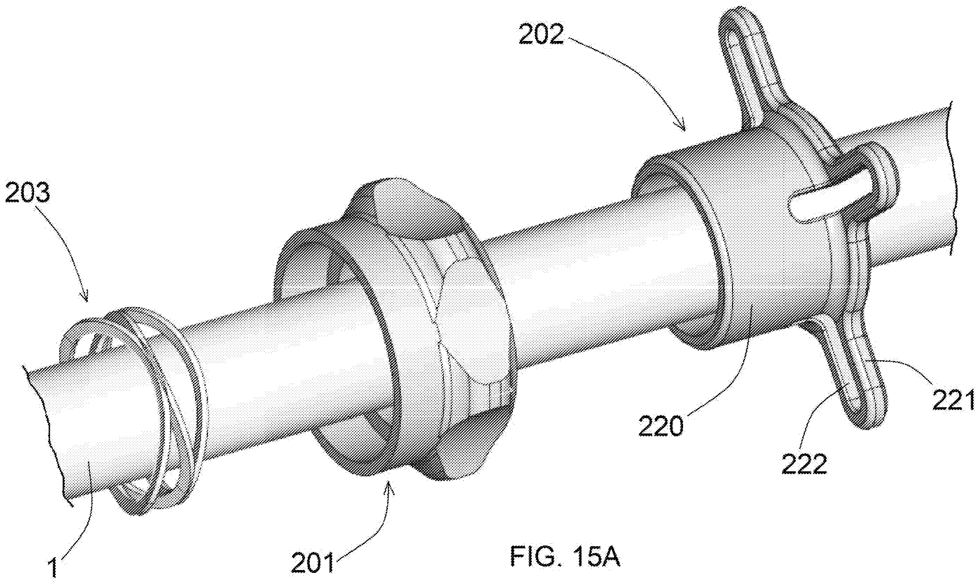

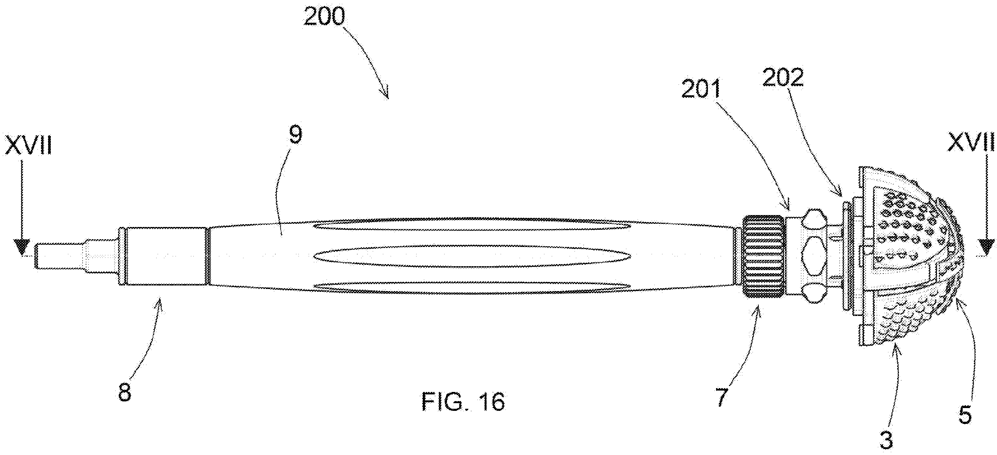

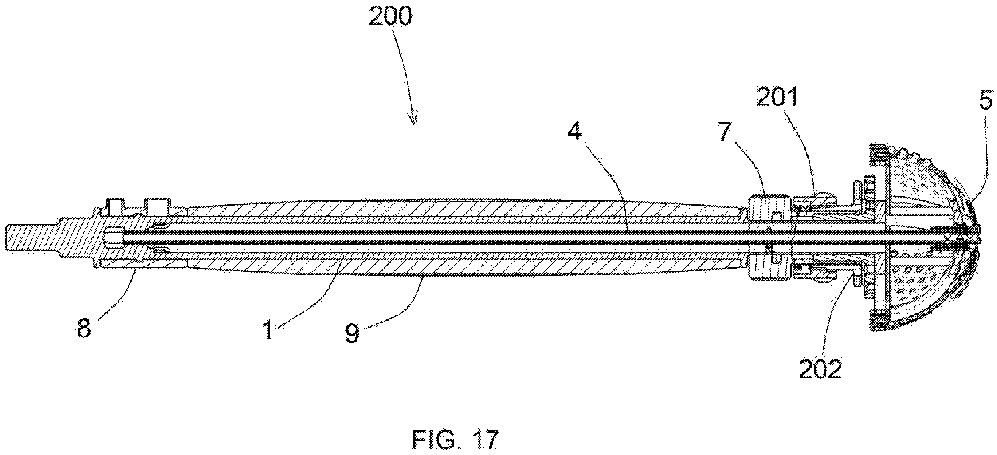

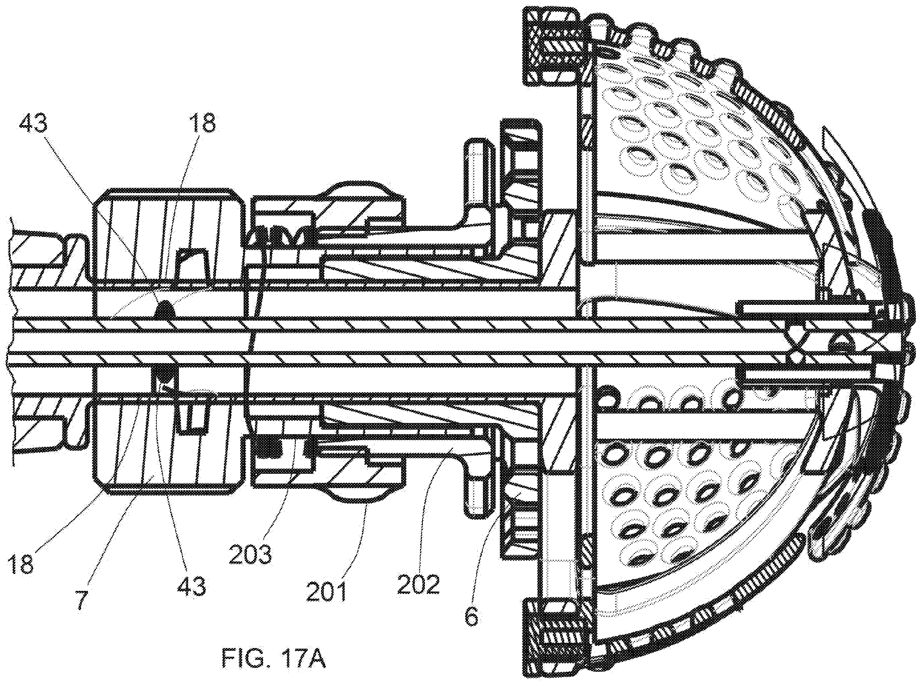

With reference to Figs. 15 - 18, a second embodiment of a milling cutter is described, indicated collectively with reference numeral (200).

The milling cutter (200) includes a locking device (202) that locks the head portions (30) in place and a locking knob (201) that locks the locking device (202). The metal ring (7) has a body (77) and a tang (78) with a smaller diameter than the body (77) of the metal ring.

Referring to Fig. 15A, the locking device (202) includes a sleeve (220) that is keyed on the shaft (1) near the cam (6) and on the tang (78) of the metal ring so that it can rotate freely. Flanges (221), which are provided with slots (222) arranged in correspondence with the slots (65) of the cam (6), protrude radially from the sleeve (220).

In this way, the second pivoting pins (37) of the head portions can pass through the slots (65) of the cam and enter the slots (222) of the flanges of the locking device.

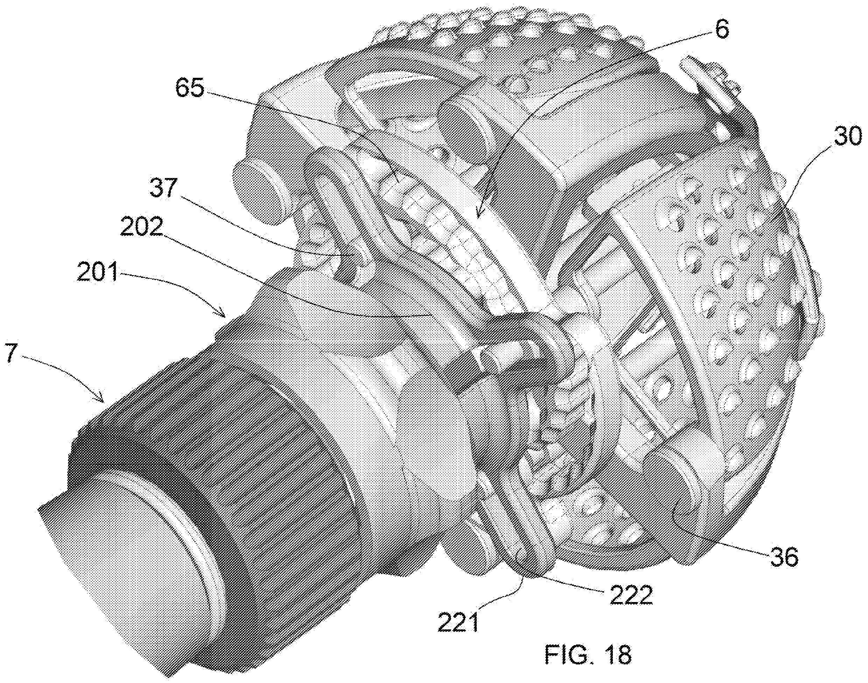

With reference to Fig. 18, initially, when the user selects the desired size of the milling cutter by turning the metal ring (7), the locking device (202) is free to rotate on the shaft (1), following the movement of the cam (6). Once the desired size of the milling cutter is set, the locking knob (201) is screwed onto the locking device (202) in such a way to lock it in rotation. In this way, since the second pivoting pins (37) of the head portions are engaged in the slots (222) of the flanges of the locking device, the head portions (30) are locked firmly in place and the milling cutter can be used.

A spring (203) is arranged around the shaft (1) between the locking knob (201) and the body (77) of the metal ring, so as to push the locking knob (201) toward the locking device (202) to ensure a secure locking of the locking device

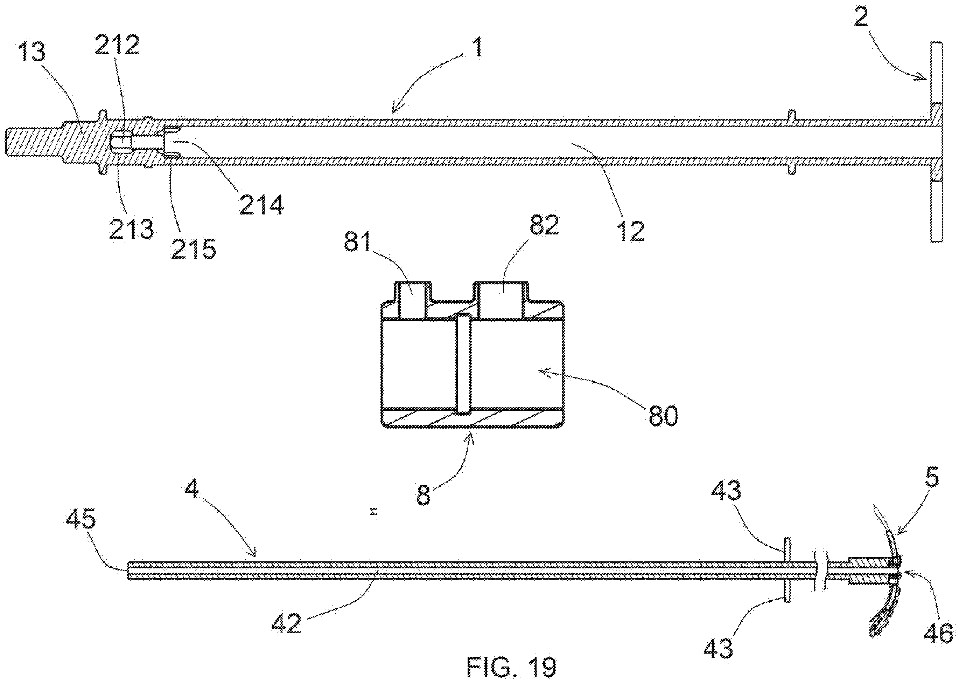

(202). Referring to Fig. 19, the shaft (1) has an axial channel (12) that extends to an end portion (212) near the coupling (13). First radial holes (213) are provided in the end portion (212) of the channel. The channel (12) has an intermediate portion (214) close to the end portion (212). Second radial holes (19a) are provided in the intermediate portion (214) of the channel.

In this case, the coupling (8) has an axial channel (80) communicating with a first radial conduit (81) and a second radial conduit (82). The coupling (80) is arranged on the shaft (1) so that the first and second radial conduit (81, 82) of the coupling communicate with the first radial holes (213) and second radial holes (215) of the shaft, respectively.

The stem (4) is arranged in the channel (12) of the shaft so that a rear end (45) of the stem is disposed in the end portion (212) of the channel of the shaft.

The first radial port (81) of the coupling is connected to a supply device of washing fluid (such as water) and the second radial conduit (82) of the coupling is connected to an extraction device.

In this way, washing fluid can be fed during operation, entering the axial channel (42) of the stem and exiting from an anterior end (46) of the stem, thus washing the bone site machined by the head of the milling cutter. Simultaneously with the supply of washing fluid, air is extracted from the cavity between the stem (4) and the shaft (1) towards the second conduit (82) of the coupling. Therefore the washing fluid mixed with bone fragments cut by the milling cutter can pass between the head portions (30) and is extracted in the cavity between the stem (4) and the shaft (1) towards the second conduit (82) of the coupling.

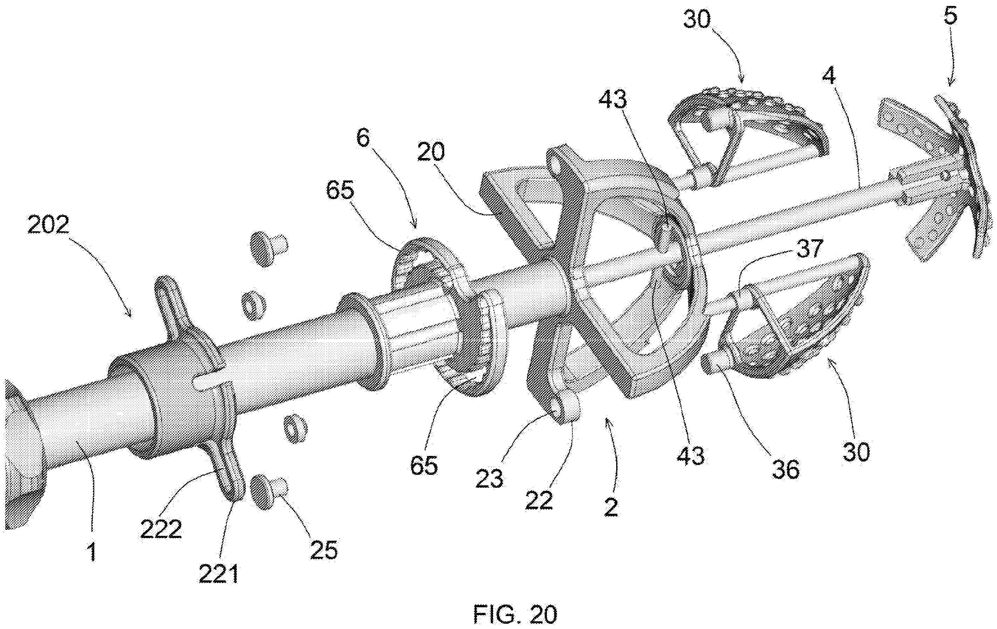

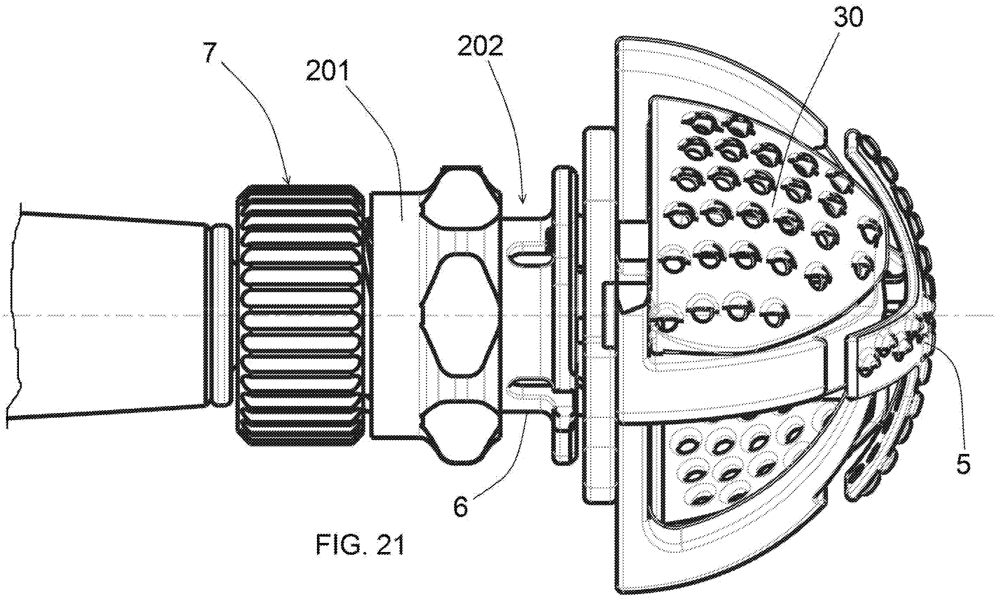

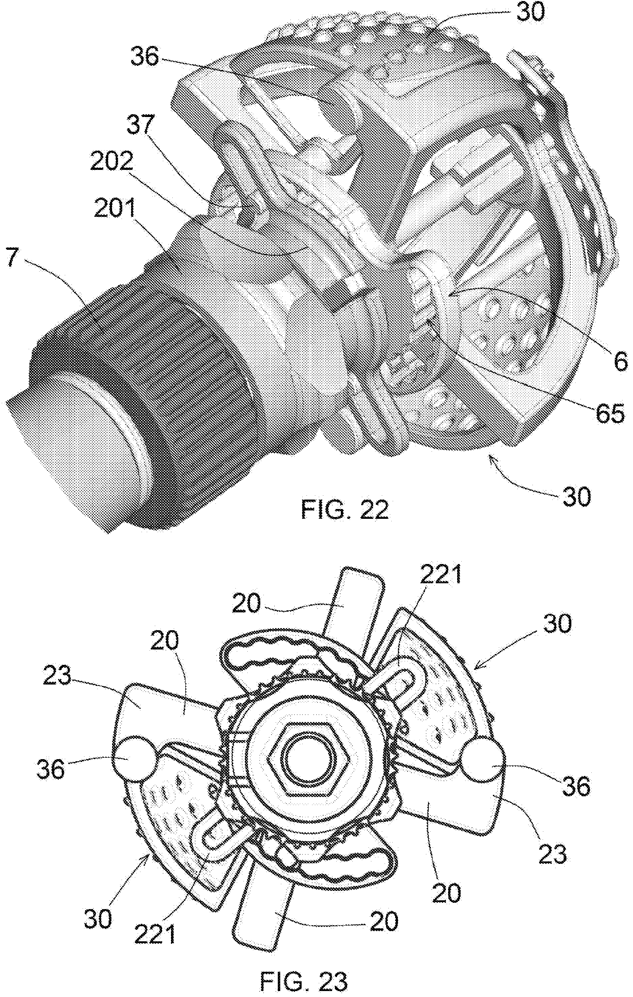

Figs. 20 to 23 illustrate a variant of the milling cutter (200) wherein only two head portions are used. In such a case, the frame (2) has only two flanges (22) with holes (23) to accommodate the second pivoting pins (37) of the head portions.

The cam (6) has two slots (65) to receive the two second pivoting pins (37) of the two head portions.

The locking device (202) has two flanges (221) that protrude radially in diametrically opposite directions. The flanges (221) of the locking device have respective slots (222) that accommodate the two second pivoting pins (37) of the two head portions that pass through the slots (65) of the cam.

The scope of the invention is expressed by the appended claims.

## Claims

1. Milling cutter (100; 200) comprising:
- a shaft (1) comprising a frame (2),
- a head (3), in the shape of a spherical cap, comprising a plurality of portions (30) movably mounted on the frame (2) so as to move radially with respect to the shaft (1) in order to go from a minimum diameter position of the head to a maximum diameter position of the head,
- a stem (4) comprising a tip (5) disposed between the portions (30) of the head; said stem (4) being mounted in such a way to slide in axial direction in the shaft (1), so that the tip (5) of the stem can go from a retracted position to a forward position
- a cam (6) movably mounted on the shaft (1) and connected to the portions (30) of the head, and
- a metal ring (7) revolvingly mounted on the shaft (1) and connected to the stem (4) and to the cam (6), in such a way that a rotation of the metal ring (7) in clockwise/counter-clockwise direction determines an increase/decrease in the diameter of the head and a forward/backward movement of the tip (5) of the stem.

2. The milling cutter (100; 200) of claim 1, wherein said cam (6) is revolvingly mounted on said shaft (1) and is coupled to said metal ring (7) in splined coupling mode in such a way to rotate together with the metal ring.

3. The milling cutter (100; 200) according to any one of the preceding claims, wherein said stem (4) is coupled inside said shaft (1), in splined coupling mode, in such a way that the stem cannot rotate with respect to the shaft.

4. The milling cutter (100; 200) according to any one of the preceding claims, wherein said stem (4) comprises two pins (43) radially projecting outwards, passing through two slots (18) of the shaft and engaging in cam grooves (74) of the metal ring, having a helical, thread-like form.

5. The milling cutter (100; 200) according to any one of the preceding claims, wherein each head portion (30) comprises:
- a side wall (31), in the form of a spherical cap portion, provided with abrasive projections (32) suitable for milling the bone, and a rear edge (33), in the form of an arc, having a first end (33a) and a second end (33b),
- a first pivoting pin (36) projecting rearwardly from the first end (33a) of the rear edge of the side wall of the head portion, for being pivoted to said frame (2) of the shaft,
- a first rod (34) radially projecting from said first end (33a) of the rear edge of the side wall of the head portion, and
- a second pivoting pin (37) projecting rearwardly from an end (34a) of the first rod (34), for being pivoted to said cam (6).

6. The milling cutter (100; 200) of claim 5, wherein each head portion (30) comprises:
- a second rod (35) radially projecting from said second end (33b) of the rear edge of the side wall of said head portion and connected to said end (34a) of the first rod, and
- a stiffening shaft (38) connected to said second pivoting pin (37) and to a front end (39) of said head portion.

7. The milling cutter (100; 200) according to claim 5 or 6, wherein said frame (2) comprises a plurality of rods (20) radially projecting from said shaft, each rod (20) of the frame having a flange (22) with a hole (23) wherein said first pivoting pin (36) of the head portion is pivoted.

8. The milling cutter (100; 200) according to any one of claims 5 to 7, wherein said cam (6) comprises a cylindrical body (60) suitable for being inserted on the shaft (1) and a plate (64) comprising a plurality of slots (65) in the shape of an arc of a circle; each slot (65) comprising a plurality of housings (66), sequentially arranged from a proximal housing (66a) proximal to the cylindrical body (60) of the cam to a distal housing (66n) distant from the cylindrical body (60) of the cam; wherein said second pivoting pin (37) of each head portion is suitable for being sequentially engaged in said housings (66) of the cam.

9. The milling cutter (100; 200) according to any one of the preceding claims, wherein said metal ring (7) comprises an indexing mechanism (75) that is coupled with the shaft (1) to allow a step-indexed rotation of the metal ring (7) relative to the shaft (1).

10. The milling cutter (200) according to any one of claims 5 to 9, comprising a locking device (202) which locks the head portions (30) in position and a locking knob (201) which locks the locking device (202); wherein said locking device (202) comprises a sleeve (220) which is inserted on the shaft (1) close to the cam (6) and flanges (221) provided with slots (222) which radially protrude from the body of the locking device in order to be arranged in correspondence with the slots (65) of the cam (6), so that said second pivoting pins (37) of the head portions pass through the slots (65) of the cam and enter the slots (222) of the flanges of the locking device.

11. The milling cutter (100) according to any one of claims 1 to 9, wherein said shaft (1) has an internally hollow front portion (11) with an axial channel (12); said front portion (11) of the shaft comprising a perforated rear portion (11a), an intermediate portion (11b) and a perforated front portion (11c);
said milling cutter comprising a coupling (8) mounted on said perforated rear portion (11a) and connected to an air inlet for extracting air from said perforated front portion (11c);
said stem (4) being disposed in said axial channel (12) of the shaft, and having an axial channel (42) open in the front and in the back in order to extract air from the tip (5) of the stem.

12. The milling cutter (100) according to any one of claims 1 to 10, wherein the shaft (1) comprises:
- an axial channel (12) open at the front and extending up to an end portion (212):
- first radial holes (213) provided in the end portion (212) of the channel,
- an intermediate portion (214) of the channel close to the end portion (212), and
- second radial holes (19a) provided in the intermediate portion (214) of the channel;
the milling cutter comprises a coupling (8) with an axial channel (80) in communication with a first radial delivery conduit (81) suitable for being connected to a washing fluid supply device, and a second radial extraction conduit (82) suitable for being connected to an extraction device; the coupling (80) is disposed on the shaft (1) in such a way that the first and second radial conduits (81, 82) of the coupling are in communication with the first radial holes (213) and the second radial holes (215) of the shaft, respectively;
the stem (4) has an axial channel (42) open at a rear end and at a front end (45, 46) and is disposed inside the channel (12) of the shaft in such a way that the rear end (45) of the stem is situated in the end portion (212) of the channel of the shaft.

## Patentansprüche

1. Fräser (100; 200), umfassend:
- eine Welle (1), umfassend einen Rahmen (2),
- einen Kopf (3) in Form einer Kugelkappe, umfassend eine Vielzahl von Abschnitten (30), die beweglich auf dem Rahmen (2) montiert sind, so dass sie sich radial in Bezug auf die Welle (1) bewegen, um von einer minimalen Umfangsposition des Kopfes zu einer maximalen Umfangsposition des Kopfes zu gelangen,
- einen Schaft (4), umfassend eine Spitze (5), die zwischen den Abschnitten (30) des Kopfes angeordnet ist; wobei der Schaft (4) derart montiert ist, dass er in axialer Richtung in der Welle (1) gleitet, so dass die Spitze (5) des Schaftes von einer zurückgezogenen Position in eine vorgeschobene Position gelangen kann;
- einen Nocken (6), der beweglich auf der Welle (1) montiert und mit den Abschnitten (30) des Kopfes verbunden ist, und
- einen Metallring (7), der drehbar auf der Welle (1) montiert und mit dem Schaft (4) und dem Nocken (6) derart verbunden ist, dass eine Drehung des Metallrings (7) in Uhrzeiger-/Gegenuhrzeigerrichtung eine Erhöhung/Verminderung des Umfangs des Kopfes und eine Vorwärts- /Rückwärtsbewegung der Spitze (5) des Schaftes bewirkt.

2. Fräser (100; 200) nach Anspruch 1, wobei der Nocken (6) drehbar auf der Welle (1) montiert und mit dem Metallring (7) im Keilwellenkopplungsmodus derart gekoppelt ist, dass er sich zusammen mit dem Metallring dreht.

3. Fräser (100; 200) nach einem der vorstehenden Ansprüche, wobei der Schaft (4) im Keilwellenmodus im Innern der Welle (1) derart gekoppelt ist, dass der Schaft sich nicht in Bezug auf die Welle drehen kann.

4. Fräser (100; 200) nach einem der vorstehenden Ansprüche, wobei der Schaft (4) zwei Stifte (43) umfasst, die radial nach außen vorstehen und durch zwei Schlitze (18) der Welle hindurchgehen und schraubenförmige, gewindeartige Nockenrillen (74) des Metallrings in Eingriff nehmen.

5. Fräser (100; 200) nach einem der vorstehenden Ansprüche, wobei jeder Kopfabschnitt (30) umfasst:
- eine Seitenwand (31) in Form eines Kugelkappenabschnitts mit abrasiven Vorsprüngen (32), die zum Fräsen des Knochens geeignet sind, und einer hinteren, bogenförmigen Kante (33), die ein erstes Ende (33a) und ein zweites Ende (33b) aufweist,
- einen ersten Drehzapfen (36), der aus dem ersten Ende (33a) der hinteren Kante der Seitenwand des Kopfabschnitts nach hinten vorsteht, um an dem Rahmen (2) der Welle verschwenkt zu werden,
- eine erste Stange (34), die radial aus dem ersten Ende (33a) der hinteren Kante der Seitenwand des Kopfabschnitts vorsteht, und
- einen zweiten Drehzapfen (37), der aus einem Ende (34a) der ersten Stange (34) nach hinten vorsteht, um an dem Nocken (6) verschwenkt zu werden.

6. Fräser (100; 200) nach Anspruch 5, wobei jeder Kopfabschnitt (30) umfasst:
- eine zweite Stange (35), die radial aus dem zweiten Ende (33b) der hinteren Kante der Seitenwand des Kopfabschnitts vorsteht und mit dem Ende (34a) der ersten Stange verbunden ist, und
- eine Versteifungswelle (38), die mit dem zweiten Drehzapfen (37) und einem vorderen Ende (39) des Kopfabschnitts verbunden ist.

7. Fräser (100; 200) nach Anspruch 5 oder 6, wobei der Rahmen (2) eine Vielzahl von Stangen (20) umfasst, die radial aus der Welle vorstehen; wobei jede Stange (20) des Rahmens einen Flansch (22) mit einem Loch (23) aufweist, in dem der erste Drehzapfen (36) des Kopfabschnitts verschwenkt ist.

8. Fräser (100; 200) nach einem der Ansprüche 5 bis 7, wobei der Nocken (6) einen zylindrischen Körper (60) umfasst, der dazu geeignet ist, auf die Welle (1) aufgezogen zu werden, und eine Platte (64), umfassend eine Vielzahl von kreisbogenförmigen Schlitzen (65); wobei jeder Schlitz (65) eine Vielzahl von Gehäusen (66) umfasst, die hintereinander von einem proximalen Gehäuse (66a) nahe dem zylindrischen Körper (60) des Nockens zu einem distalen Gehäuse (66n) entfernt von dem zylindrischen Körper (60) des Nockens angeordnet sind; wobei der zweite Drehzapfen (37) eines jeden Kopfabschnitts dazu geeignet ist, die Gehäuse (66) des Nockens hintereinander in Eingriff zu nehmen.

9. Fräser (100; 200) nach einem der vorstehenden Ansprüche, wobei der Metallring (7) einen Indexierungsmechanismus (75) umfasst, der mit der Welle (1) gekoppelt ist, um eine stufenweise indexierte Drehung des Metallrings (7) in Bezug auf die Welle (1) zu erlauben.

10. Fräser (200) nach einem der Ansprüche 5 bis 9, umfassend eine Blockiervorrichtung (202), die die Position der Kopfabschnitte (30) blockiert, und einen Blockierknopf (201), der die Blockiervorrichtung (202) blockiert; wobei die Blockiervorrichtung (202) eine Hülse (220) umfasst, die auf die Welle (1) nahe dem Nocken (6) aufgezogen ist, und Flansche (221), die mit Schlitzen (222) versehen ist, die radial aus dem Körper der Blockiervorrichtung vorstehen, um in Übereinstimmung mit den Schlitzen (65) des Nockens (6) angeordnet zu werden, so dass die zweiten Drehzapfen (37) der Kopfabschnitte durch die Schlitze (65) des Nockens hindurchgehen und in die Schlitze (222) der Flansche der Blockiervorrichtung eindringen.

11. Fräser (100) nach einem der Ansprüche 1 bis 9, wobei die Welle (1) einen vorderen, inwendig hohlen Abschnitt (11) mit einem axialen Kanal (12) aufweist; wobei der vordere Abschnitt (11) der Welle einen gelochten hinteren Abschnitt (11a), einen Zwischenabschnitt (11b) und einen gelochten vorderen Abschnitt (11c) umfasst;
wobei der Fräser eine Kupplung (8) umfasst, die auf den gelochten hinteren Abschnitt (11a) montiert und mit einem Lufteinlass verbunden ist, um Luft aus dem gelochten vorderen Abschnitt (11c) abzusaugen;
wobei der Schaft (4) in dem axialen Kanal (12) der Welle angeordnet ist und einen axialen Kanal (42) aufweist, der vorn und hinten offen ist, um Luft aus der Spitze (5) des Schafts abzusaugen.

12. Fräser (100) nach einem der Ansprüche 1 bis 10, wobei die Welle (1) umfasst:
- einen axialen Kanal (12), der vorne offen ist und sich bis zu einem Endabschnitt (212) erstreckt:
- erste radiale Löcher (213), die im Endabschnitt (212) des Kanals vorgesehen sind,
- einen Zwischenabschnitt (214) des Kanals, nahe dem Endabschnitt (212), und
- zweite radiale Löcher (19a), die im Zwischenabschnitt (214) des Kanals vorgesehen sind;
der Fräser umfasst eine Kupplung (8) mit einem axialen Kanal (80), der mit einer ersten radialen Förderleitung (81) in Verbindung steht, die dazu geeignet ist, mit einer Vorrichtung zur Zuführung von Spülflüssigkeit verbunden zu werden, und einer zweiten radialen Saugleitung (82), die dazu geeignet ist, mit einer Saugvorrichtung verbunden zu werden; die Kupplung (80) ist auf der Welle (1) derart angeordnet, dass die erste und die zweite radiale Leitung (81, 82) der Kupplung jeweils mit den ersten radialen Löchern (213) und den zweiten radialen Löchern (215) der Welle in Verbindung stehen;
der Schaft (4) weist einen axialen Kanal (42) auf, der an einem vorderen und an einem hinteren Ende (45, 46) offen ist und in dem Kanal (12) der Welle derart angeordnet ist, dass das hintere Ende (45) des Schafts sich in dem Endabschnitt (212) des Kanals der Welle befindet.

## Revendications

1. Fraise (100; 200) comprenant :
- un arbre (1) comprenant un châssis (2),
- une tête (3), en forme de calotte sphérique, comprenant une pluralité de portions (30) montées mobiles sur le châssis (2) de manière à se déplacer radialement par rapport à l'arbre (1) pour passer d'une position de diamètre minimum de la tête à une position de diamètre maximum de la tête,
- une tige (4) comprenant une pointe (5) disposée entre les portions (30) de la tête ; ladite tige (4) étant montée coulissante axialement dans l'arbre (1) de façon que la pointe (5) de la tige puisse passer d'une position rétractée à une position avancée,
- une came (6) montée mobile sur l'arbre (1) et reliée aux portions (30) de la tête, et
- une frette (7) montée pivotante sur l'arbre (1) et reliée à la tige (4) et à la came (6), de manière qu'une rotation de la frette (7) dans le sens horaire/antihoraire provoque une augmentation/diminution du diamètre de la tête et un avancement/recul de la pointe (5) de la tige.

2. Fraise (100; 200) selon la revendication 1, où ladite came (6) est montée pivotante sur ledit arbre (1) et elle est couplée à ladite frette (7), en relation d'accouplement rainuré, de façon à tourner avec la frette.

3. Fraise (100; 200) selon l'une quelconque des revendications précédentes, où ladite tige (4) est couplée dans ledit arbre (1), en relation d'accouplement rainuré, de façon que la tige ne puisse pas tourner par rapport à l'arbre.

4. Fraise (100; 200) selon l'une quelconque des revendications précédentes, où ladite tige (4) comprend deux pions (43) qui débordent radialement vers l'externe, en traversant deux fissures (18) de l'arbre et en s'engageant dans des rainures à came (74) de la frette, ayant une forme hélicoïdale, du type filetage.

5. Fraise (100; 200) selon l'une quelconque des revendications précédentes, où chaque portion (30) de la tête comprend :
- une paroi latérale (31), en forme de portion de calotte sphérique, ayant des saillies abrasives (32) aptes à fraiser l'os et un bord postérieur (33), en forme d'arc, ayant une première extrémité (33a) et une seconde extrémité (33b),
- un premier pivot de pivotement (36) qui déborde postérieurement de la première extrémité (33a) du bord postérieur de la paroi latérale de la portion de tête, pour se pivoter au susdit châssis (2) de l'arbre,
- une première tige (34) qui déborde radialement de ladite première extrémité (33a) du bord postérieur de la paroi latérale de la portion de tête, et
- un second pivot de pivotement (37) qui déborde postérieurement d'une extrémité (34a) de la première tige (34), pour se pivoter à ladite came (6).

6. Fraise (100; 200) selon la revendication 5, où chaque portion (30) de tête comprend :
- une seconde tige (35) qui déborde radialement de ladite seconde extrémité (33b) du bord postérieur de la paroi latérale de la portion de tête et reliée à ladite extrémité (34a) de la première tige, et
- un arbre de raidissement (38) relié au susdit second pivot de pivotement (37) et à une extrémité antérieure (39) de ladite portion de tête.

7. Fraise (100; 200) selon la revendication 5 ou 6, où ledit châssis (2) comprend une pluralité de tiges (20) qui débordent radialement du susdit arbre ; chaque tige (20) du châssis ayant une bride (22) avec un orifice (23) dans lequel ledit premier pivot de pivotement (36) de la portion de tête est pivoté.

8. Fraise (100; 200) selon l'une quelconque des revendications de 5 à 7, où ladite came (6) comprend un corps cylindrique (60) apte à être inséré sur l'arbre (1) et une plaque (64) comprenant une pluralité de fissures (65) en forme d'arc de cercle ; chaque fissure (65) comprenant une pluralité d'emplacements (66), disposés en séquence depuis un emplacement proximal (66a) proche du corps cylindrique (60) de la came à un emplacement distal (66n) éloigné du corps cylindrique (60) de la came ; où ledit second pivot de pivotement (37) de chaque portion de tête est apte à s'engager séquentiellement dans lesdits emplacements (66) de la came.

9. Fraise (100; 200) selon l'une quelconque des revendications précédentes, où ladite frette (7) comprend un mécanisme d'indexation (75) qui s'accouple avec l'arbre (1) pour permettre une rotation indexée à déclics de la frette (7) par rapport à l'arbre (1).

10. Fraise (200) selon l'une quelconque des revendications de 5 à 9, comprenant un dispositif de blocage (202) qui bloque en position les portions de tête (30) et une molette de verrouillage (201) qui bloque le dispositif de blocage (202) ; où ledit dispositif de blocage (202) comprend un manchon (220) qui est inséré sur l'arbre (1) à proximité de la came (6) et des frettes (221) munies de fissures (222) en forme de fente qui débordent radialement du corps du dispositif de blocage pour se disposer en correspondance des fissures (65) de la came (6), de manière que lesdits seconds pivots de pivotement (37) des portions de tête traversent les fissures (65) de la came et entrent dans les fissures (222) des frettes du dispositif de blocage.

11. Fraise (100) selon l'une quelconque des revendications de 1 à 9, où l'arbre (1) a une portion antérieure (11) internement creuse avec un canal axial (12) ; ladite portion antérieure (11) de l'arbre comprenant une portion postérieure perforée (11a), une portion intermède (11b) et une portion antérieure perforée (11c) ;
ladite fraise comprenant un raccord (8) monté sur ladite portion postérieure perforée (11a) et relié à une aspiration d'air, pour aspirer l'air de ladite portion antérieure perforée (11c) ;
ladite tige (4) étant disposée dans ledit canal axial (12) de l'arbre et ayant un canal axial (42) ouvert antérieurement et postérieurement pour aspirer l'air de la pointe (5) de la tige.

12. Fraise (100) selon l'une quelconque des revendications de 1 à 10, où l'arbre (1) comprend :
- un canal axial (12) ouvert antérieurement et qui se déploie jusqu'à une portion d'extrémité (212) :
- des premiers orifices radiaux (213) prévus dans la portion d'extrémité (212) du canal,
- une portion intermède (214) du canal proche de la portion d'extrémité (212), et
- des seconds orifices radiaux (19a) prévus dans la portion intermède (214) du canal ;
la fraise comprend un raccord (8) ayant un canal axial (80) en communication avec un premier conduit radial (81) d'amenée destiné à être relié à un dispositif d'alimentation fluide de lavage et un second conduit radial (82) d'aspiration destiné à être relié à un dispositif d'aspiration ; le raccord (80) est disposé sur l'arbre (1) de manière que le premier et le second conduit radial (81, 82) du raccord communiquent respectivement avec les premiers orifices radiaux (213) et les seconds orifices radiaux (215) de l'arbre ;
la tige (4) a un canal axial (42) ouvert à une extrémité postérieure et antérieure (45, 46) et elle est disposée dans le canal (12) de l'arbre de manière que l'extrémité postérieure (45) de la tige se trouve dans la portion d'extrémité (212) du canal de l'arbre.
